# EUROPÄISCHE PATENTSCHRIFT

(11) **EP 2 563 224 B1**
(45) Veröffentlichungstag und Bekanntmachung des Hinweises auf die Patenterteilung: **23.03.2016**
(21) Anmeldenummer: 11721231.6
(22) Anmeldetag: 21.04.2011
(51) Int. Cl.: A61B 6/00

(54) **RÖNTGENSYSTEM UND VERFAHREN ZUR GENERIERUNG VON 3D-BILDDATEN**
X-RAY SYSTEM AND METHOD FOR GENERATING 3D IMAGE DATA
SYSTÈME À RAYONS X ET PROCÉDÉ DE GÉNÉRATION DE DONNÉES D'IMAGE 3D

(30) Priorität: 26.04.2010 DE 102010018627
(43) Veröffentlichungstag der Anmeldung: 06.03.2013
(73) Patentinhaber: Fraunhofer-Gesellschaft zur Förderung der angewandten Forschung e.V., 80686 München (DE); Charité Universitätsmedizin Berlin, 10117 Berlin (DE)
(72) Erfinder: KEEVE, Erwin, 14469 Potsdam (DE); STOPP, Fabian, 10249 Berlin (DE); UHLMANN, Eckart, 25368 Kiebitzreihe (DE)
(74) Vertreter: Pfenning, Meinig & Partner mbB
(86) Internationale Anmeldenummer: PCT/EP2011/002167
(87) Internationale Veröffentlichungsnummer: WO 2011/134676

(56) Entgegenhaltungen:
- US-A1- 2004 170 254
- US-A1- 2008 013 692

## Beschreibung

Die vorliegende Erfindung betrifft ein Röntgensystem sowie ein entsprechendes Verfahren zur Generierung von 3D-Bilddaten.

Röntgensysteme dienen in der Medizin der Untersuchung von Patienten mittels Röntgenstrahlung, wobei derartige Systeme eine Röntgenquelle und einen Röntgendetektor aufweisen. In den Materialwissenschaften werden zu untersuchende Objekte ebenfalls mit Röntgenstrahlung durchleuchtet, um zerstörungsfrei Informationen über den inneren Aufbau zu erhalten. Die von der Röntgenquelle erzeugten Röntgenstrahlen durchdringen dabei das abzubildende Gewebe bzw. Material und werden je nach Gewebetyp oder Materialtyp unterschiedlich stark abgeschwächt. Am Röntgenbilddetektor einer Röntgenvorrichtung entsteht ein Projektionsbild des durchleuchteten Objekts, bei dem räumliche Informationen überlagert dargestellt werden. Erst eine dreidimensionale, genaue Darstellung des Körperinnern erlaubt jedoch beispielsweise eine exakte Reposition von Knochenbrüchen an Gelenken oder eine genaue Positionierung von Implantaten relativ zu kritischen anatomischen Strukturen.

Zur Erzeugung dreidimensionaler Bilddaten werden mit einer Röntgenvorrichtung mehrere zweidimensionale Projektionsaufnahmen eines Objekts aus verschiedenen Raumrichtungen aufgenommen und anschließend über einen Algorithmus das gescannte Volumen rekonstruiert. Neben den 2D-Projektionsbildern benötigt der Algorithmus dafür Lageinformationen von Röntgenstrahler und Strahlendetektor, weiter muss berücksichtigt werden, dass das zu rekonstruierende Objekt stets in den Projektionsaufnahmen abgebildet wird.

Aus dem Stand der Technik sind bereits mehrere Verfahren und Vorrichtungen zur Erzeugung dreidimensionaler Röntgenbilder bekannt. Oftmals werden hierzu sogenannte "C-Bögen" verwendet, wie beispielsweise in WO 2010/012441 A1 beschrieben. Diese C-Bögen erlauben zwar eine zuverlässige Rekonstruktion des gescannten Bereichs, die Bildqualität ist für intraoperative Zwecke ausreichend, bedingt durch ihre Bauform hat ein Chirurg bei diesen Geräten aber keinen unmittelbaren Zugang zum kompletten Körper des Patienten.

Computertomographen ermöglichen eine sehr gute Bildqualität, sind allerdings ungeeignet für den intraoperativen Einsatz, da hier der Zugang zum Patienten durch das Gerät versperrt ist. Zusätzlich benötigen derartige Geräte viel Platz, haben eine hohe Strahlenexposition, sind komplex in der Bedienung und verursachen hohe Kosten.

Der vorliegenden Erfindung liegt damit die Aufgabe zugrunde, ein Röntgensystem zu entwickeln, das die genannten Nachteile vermeidet, mit dem also bei geringer Strahlenexposition reproduzierbare 3D-Röntgenaufnahmen erzeugt werden können, während das abzubildende Objekt nicht vom Gerät umschlossen wird und wobei die Aufnahmen in beliebigen Positionen gemacht werden können.

Diese Aufgabe wird erfindungsgemäß gelöst durch ein Röntgensystem und ein Verfahren zu dessen Bedienung.

Das Röntgensystem umfasst einen Strahlendetektor und einen beweglichen Röntgenstrahler, die zur Bildgebung benötigt werden, sowie eine Steuereinheit zum Bedienen des Systems. Der Strahlendetektor ist hierbei in einem Arbeitszustand des Systems raumfest positioniert, allerdings aus dieser Position lösbar. Somit kann der Strahlendetektor an mehreren Stellen angebracht werden, was zusammen mit der Beweglichkeit des Röntgenstrahlers die Variabilität des Systems erhöht. Der raumfest positionierte Strahlendetektor definiert ein ortsfestes Koordinatensystem, zur Aufnahme von Röntgenprojektionsbildern bewegt sich der Röntgenstrahler relativ zum Strahlendetektor in unterschiedliche Relativpositionen. Der Röntgenstrahler definiert durch seine Position und Orientierung ein durch eine Transformation in das ortsfeste Koordinatensystem überführbares körperfestes Bezugskoordinatensystem bezüglich des ortsfesten Basiskoordinatensystems, sodass die Relativpositionen in beiden Koordinatensystemen eindeutig definiert sind. Aus den in diesen Relativpositionen aufgenommenen Röntgenprojektionsbildern und den Koordinaten der Relativpositionen lassen sich durch eine iterative Rekonstruktionstechnik oder andere Bildverarbeitungsmethoden 3D-Röntgenaufnahmen berechnen.

Zum mobilen und intraoperativen Einsatz umfasst das System ein bewegliches Stativ mit einem motorisch beweglichen Stativarm und einem Stativfuß. Der Röntgenstrahler ist am Stativarm befestigt und kann in mindestens vier Freiheitsgraden bewegt werden. Diese vier Freiheitsgrade sind notwendig, um Röntgenprojektionsbilder mit der nötigen Informationstiefe zur Rekonstruktion einer dreidimensionalen Darstellung zu erhalten. Diese Freiheitsgrade umfassen vorzugsweise drei translatorische Freiheitsgrade, um das komplette abzubildende Objekt scannen zu können und den Scanvorgang in verschiedenen Höhen über dem Objekt durchzuführen. Zusätzlich ist in diesem Fall ein rotatorischer Freiheitsgrad notwendig, um den Röntgenstrahler in den verschiedenen durch translatorische Bewegung erreichbaren Positionen in Richtung des abzubildenden Volumens des Objekts drehen zu können und so dieses Volumen unter einem anderen Winkel zu erfassen. Entscheidend dabei ist, dass der Röntgenstrahler immer auf den Strahlendetektor gerichtet bleibt. Alternativ dazu kann beispielsweise auch nur eine translatorische Bewegung mit zwei Freiheitsgraden in einer Ebene parallel zum Strahlendetektor und eine Verkippung des Röntgenstrahlers um zwei rotatorische Freiheitsgrade, also um zwei Winkel, vorgesehen sein.

Der Stativfuß wird über Befestigungsvorrichtungen ebenfalls raumfest positioniert und weist somit bezüglich des Strahlendetektors einen festen Abstand und eine feste Position innerhalb des ortsfesten Basiskoordinatensystems auf. Das Stativ erlaubt eine freie Positionierung des Systems, sodass ein Arzt auch während einer Operation noch Zugang zum Körper des Patienten hat. Der Stativarm ist ebenfalls frei beweglich und kann bei Operationen einfach aus dem Arbeitsbereich des Arztes entfernt und für neue Aufnahmen wieder in eine Aufnahmeposition gebracht werden. Die Gelenke des Stativarms sind mit absoluten Messwertgebern der Gelenkstellungen ausgestattet, so dass durch die motorische Ansteuerung des Stativarms reproduzierbar Positionen und Orientierungen des Röntgenstrahlers angefahren werden können. Somit können reproduzierbar Aufnahmen bestimmter Bereiche gemacht und hinsichtlich Änderungen, beispielsweise beim Einbringen eines Implantats, miteinander verglichen werden.

Vorteilhafte Weiterbildungen des Systems werden in den Unteransprüchen beschrieben.

Eine vorteilhafte Weiterbildung sieht vor, dass der Stativfuß Sensoren zur Überprüfung der Beibehaltung einer raumfesten Lage des Stativfußes umfasst. Da die 3D-Röntgenbildgebung nur zuverlässig und reproduzierbar funktioniert, wenn die Lage des Stativfußes stets raumfest in Bezug auf den Strahlendetektor ist, können Sensoren im Stativfuß beispielsweise den Abstand zu einem Patiententisch oder zu anderen raumfesten Gegenständen wie Lampen messen und bei Abweichungen vom zuvor festgelegten Abstand eine Warnung auf einer Anzeigeeinheit ausgeben.

Weiter kann das System Sensoren, beispielsweise Bewegungsmelder, umfassen, die kontrollieren, ob sich Personen an bestimmten Stellen aufhalten. Da die Strahlenexposition für alle an einer Untersuchung beteiligten Personen so gering wie möglich gehalten werden soll, können diese Sensoren detektieren, ob während einer Aufnahme noch Personen im Raum sind oder sich sogar im Aufnahmebereich des Röntgenstrahlers befinden und daraufhin die Aufnahme unterbrechen und über die Anzeigeeinheit eine Fehlermeldung ausgeben. Somit wird eine irrtümliche Bedienung des Röntgensystems ausgeschlossen. Weiter kann beispielsweise auch detektiert werden, ob ein Patient zu früh aus einer Narkose erwacht und sich aus dem Aufnahmebereich bewegt, sodass hier eine unnötige Strahlenexposition für den Patienten aufgrund überflüssiger Röntgenaufnahmen vermieden wird.

In einer vorteilhaften Weiterbildung umfassen die zuvor beschriebenen Sensoren Laser, mittels derer Abstände zwischen Objekten oder auch Bewegungen sehr genau detektiert werden können.

Der Röntgenstrahler kann auch in fünf Freiheitsgraden bewegbar sein, was eine größere Variabilität seiner Orientierung bezüglich des abzubildenden Objekts zur Folge hat. In besonders vorteilhafter Weise ist der Röntgenstrahler in sechs Freiheitsgraden, also allen drei translatorischen und allen drei rotatorischen Freiheitsgraden, bewegbar.

Eine weitere vorteilhafte Weiterbildung sieht vor, dass Lagesensoren, wie z. B. Gyroskope oder Beschleunigungssensoren, zum Ermitteln der Relativlage des Röntgenstrahlers bezüglich des Basiskoordinatensystems im Röntgensystem enthalten sind. Neben der Positionsbestimmung über die Gelenkstellungen des Stativarms existiert so eine zweite Möglichkeit der Positionsbestimmung, die die Genauigkeit der Positionsbestimmung erhöht.

Um den Stativfuß raumfest zu fixieren, können Räder, mit denen er ansonsten frei im Raum bewegt werden kann, blockiert werden oder durch Vakuumansaugen das Stativ am Boden festgehalten werden. Eine Fixierung des Stativs ist wichtig für die Rekonstruktion der dreidimensionalen Aufnahmen, da es hierbei auf eine seit der Kalibrierung nicht veränderte Relativposition zwischen Strahlendetektor und Röntgenquelle ankommt.

Eine weitere vorteilhafte Weiterbildung sieht vor, dass der Strahlendetektor durch Haltevorrichtungen raumfest lösbar positionierbar ist. Hiermit wird eine raumfeste Fixierung des Strahlendetektors an einer Vielzahl von Objekten wie OP-Tischen, Krankenliegen o. ä. erreicht, wobei die Fixierung vorzugsweise an raumfesten Objekten stattfindet, da in diesem Fall das ortsfeste Koordinatensystem über einen nicht beweglichen Fixpunkt bestimmt ist.

Der Stativarm kann neben einem motorischen Verfahren auch manuell in bestimmte Positionen einstellbar sein, beispielsweise, um in einem "Teach-in"-Verfahren eine gewünschte Aufnahmebahn von Röntgenprojektionsbildern einzustellen.

Zur Erhöhung der Stabilität des Röntgensystems kann der Strahlendetektor durch einen Adapter mechanisch mit dem Stativ verbunden sein. Damit wird stets ein exakt definierter Abstand und eine exakte Position innerhalb des ortsfesten Basiskoordinatensystems vorgegeben, die nicht irrtümlich verändert werden können.

In vorteilhafter Weiterbildung umfasst das Röntgensystem eine Positioniereinheit für das abzubildende Objekt, beispielsweise einen OP-Tisch, an der der Strahlendetektor angebracht wird, wobei der Strahlendetektor vorzugsweise eine gleiche Breite wie die verwendete Positioniereinheit aufweist (vorliegend wird unter "gleiche Breite" eine Variation der Breite von Strahlendetektor und Positioniereinheit von +/-10 % verstanden). Somit wird sichergestellt, dass das abzubildende Objekt komplett auf dem Strahlendetektor abbildbar ist.

Als Weiterbildung des vorgenannten Röntgensystems als eigenständige Erfindung (das heißt als Strahlendetektor allein, ohne zusätzliche Merkmale des vorgenannten Röntgensystems) umfasst der Strahlendetektor mehrere einzelne Detektoren, die durch Gelenke und Rastelemente zu einem klappbaren oder ausziehbaren Detektorsystem kombiniert werden können. Die Gelenke und Rastelemente stellen dabei neben einer mechanischen auch eine elektrische Verbindung zwischen den einzelnen Detektorplatten her. Alternativ können die einzelnen Detektorplatten über separate elektrische Leitungen mit dem Rest des Systems verbunden werden. Der Detektor kann so platzsparend komprimiert werden, indem die Detektorplatten aneinander angeklappt oder untereinander verschoben werden. Somit kann das zu untersuchende Objekt leichter erreicht werden, um Modifikationen daran vorzunehmen. Zur Aufnahme von weiteren Röntgenbildern wird der Strahlendetektor auf die volle Länge bzw. Breite ausgeklappt oder ausgezogen und erlaubt somit eine größere Variabilität der Röntgenaufnahmen, da durch eine vergrößerte Detektorfläche ein größerer Winkelbereich aufgenommen werden kann.

Das Verfahren zur Benutzung eines beschriebenen Röntgensystems umfasst als ersten Schritt eine Kalibrierung des Systems bei raumfest fixiertem Strahlendetektor und raumfest fixiertem Stativfuß durch einen Kalibrierkörper. Der Kalibrierkörper wird hierzu zwischen Röntgenstrahler und Strahlendetektor befestigt, steht aber vorzugsweise in unmittelbarer Verbindung mit dem Strahlendetektor. Der Kalibrierkörper enthält eine vorgegebene Struktur an Material, welches Röntgenstrahlung abschwächt, z. B. ein Metallgitter. Es wird mindestens ein Röntgenprojektionsbild des Kalibrierkörpers aufgenommen, anhand dieses Projektionsbilds bzw. der Projektionsbilder und der bekannten Geometrie des dreidimensionalen Kalibrierkörpers kann die Position und Orientierung des Strahlungsdetektors in Bezug zum durch die Position des Strahlendetektors definierten Basiskoordinatensystems bestimmt werden.

Nach der erfolgten Kalibrierung wird der Kalibrierkörper entfernt und an seine Stelle das abzubildende Objekt zwischen Röntgenstrahler und Strahlendetektor eingebracht. Vom abzubildenden Objekt werden nun mehrere Röntgenprojektionsbilder in beliebigen Relativpositionen aufgenommen, aus denen durch eine iterative Rekonstruktionstechnik durch die Koordinaten der zum Aufnehmen verwendeten Relativpositionen eine dreidimensionale Rekonstruktion des durchstrahlten Volumenbereichs erstellt wird. Durch die Verwendung einer iterativen Rekonstruktionstechnik oder anderer Bildverarbeitungsmethoden sind wenige Projektionsbildaufnahmen zur dreidimensionalen Bildgebung ausreichend und die Strahlenexposition des Patienten wird minimiert.

Eine vorteilhafte Weiterbildung des Verfahrens sieht vor, dass einem motorisch bewegbaren Arm durch ein "Teach-in"-Verfahren eine Aufnahmebahn zum Aufnehmen von Röntgenprojektionsbildern vorgegeben wird. Hierzu wird von Hand der Stativarm mit dem daran befestigten Röntgenstrahler in bestimmte Relativpositionen gefahren, in denen später die Projektionsaufnahmen gemacht werden sollen und die die Aufnahmebahn definieren. Die Koordinaten dieser Relativpositionen werden in einer Steuereinheit hinterlegt und nach Beendigung des manuellen Einstellens können durch die Steuereinheit und die Motorsteuerung des Stativarms diese Relativpositionen automatisch angefahren werden, sodass automatisiert entlang einer Aufnahmebahn Projektionsaufnahmen erstellt werden können.

Eine weitere vorteilhafte Weiterbildung des Verfahrens sieht vor, dass durch ein "Teach-in"-Verfahren eine Bewegungsbahn des Stativarms mit Röntgenstrahler zur Projektionsbildaufnahme vorgegeben wird. Mit einer definierten Anzahl an aufzunehmenden Röntgenprojektionsbildern werden anschließend automatisch die optimalen Positionen des Röntgenstrahlers auf der Aufnahmebahn berechnet. Durch die Steuereinheit und die Motorsteuerung des Stativarms wird die Aufnahmebahn des "Teach-in"-Verfahrens automatisch abgefahren und an den berechneten Positionen werden Röntgenprojektionsbilder aufgenommen.

Ausführungsbeispiele der Erfindung sind in den Zeichnungen dargestellt und werden nachfolgend anhand der Figuren 1 bis 3 erläutert.

Es zeigen:
- Fig. 1: eine schematische Darstellung des Röntgensystems mit Kalibrierkörper in Seitenansicht,
- Fig. 2: eine schematische Darstellung des Röntgensystems mit Patienten in Seitenansicht,
- Fig. 3: eine Aufsicht auf das Röntgensystem aus Fig. 2,
- Fig. 4a: einen zusammenklappbaren Strahlendetektor,
- Fig. 4b: einen ausziehbaren Strahlendetektor.

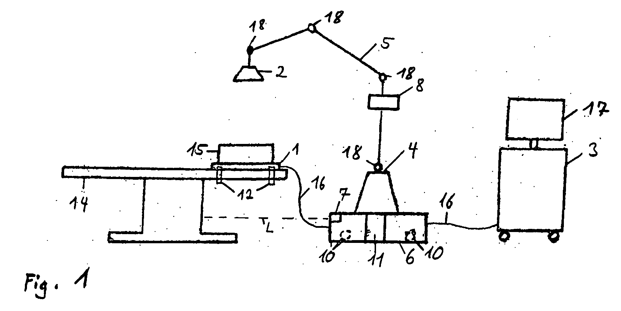

In Fig. 1 ist in einer seitlichen Ansicht ein erfindungsgemäßes System dargestellt. Der Strahlendetektor 1, der eine Szintillatorschicht enthält, die auftreffende Röntgenstrahlung in sichtbares Licht konvertiert, welches von lichtempfindlichen Elektronikelementen detektiert wird, liegt auf einer Positioniereinheit 14 für das abzubildende Objekt, in diesem Fall einem OP-Tisch aus Metall, der raumfest mit dem Boden verbunden ist, auf und ist mit Haltevorrichtungen 12, wie beispielsweise Klemmen aus Kunststoff oder Metall, an diesem befestigt. Die Haltevorrichtungen 12 können gelöst werden und der Strahlendetektor 1 somit an einer anderen Position fixiert werden. Zum einfacheren Transport und zur Befestigung sind am Strahlendetektor 1 hier nicht dargestellte Griffe vorgesehen.

Ein Stativ 4 ist raumfest positioniert, indem ein Stativfuß 6 durch ein Vakuumansaugsystem 11 am Boden gehalten wird, sodass er trotz der Räder 10 nicht mehr bewegt werden kann. Eine Bewegung des Stativfußes kann aber auch mittels Rollen oder schienengeführt erfolgen. Ein Stativarm 5 ist so eingestellt, dass sich ein Röntgenstrahler 2 über dem auf dem Strahlendetektor aufliegenden Kalibrierkörper 15 befindet. Die für den Betrieb des Röntgenstrahlers benötigten Komponenten, wie z. B. ein Hochspannungsgenerator, sind in dieser Darstellung nicht gezeigt, aber hinlänglich bekannt. Der Kalibrierkörper 15 besteht aus einem Draht- oder Kugelmodell, wobei die Drähte bzw. die einzelnen Kugeln in mehreren Ebenen versetzt zueinander angeordnet sind. In dieser Position können die zur Kalibrierung des Systems benötigten Projektionsaufnahmen erstellt werden. Zur Übermittlung des erzielten Bilds und zur Energieversorgung ist der Strahlendetektor 1 über eine elektrische Verbindung 16 mit dem Stativ 4 und dieses über eine elektrische Verbindung mit einer Steuereinheit 3 und einer Anzeigeeinheit 17 verbunden. Die Steuereinheit umfasst hier einen PC, der mit einem Bildschirm als Anzeigeeinheit 17 verbunden ist.

Über einen Sensor zur Abstandsmessung 7 wird durch einen Laserstrahl L der Abstand zwischen dem Fuß der Positioniereinheit 14 und dem Stativfuß 6 bestimmt und bei Abweichungen eine Fehlermeldung auf der Anzeigeeinheit 17 ausgegeben. Der Laserstrahl L weist eine nur schwache Intensität auf und ist nahe des Bodens geführt, um keine Personen durch Exposition zu gefährden. Zusätzlich liegt die Wellenlänge im sichtbaren Bereich, um an der Untersuchung beteiligten Personen die Position des Lasers zu verdeutlichen. Ein Bewegungsmelder 8, der einen Infrarotsensor umfasst, registriert die Anwesenheit von Personen im Raum und unterbricht die Röntgenaufnahme, sofern sich Personen in der Umgebung des Röntgensystems aufhalten.

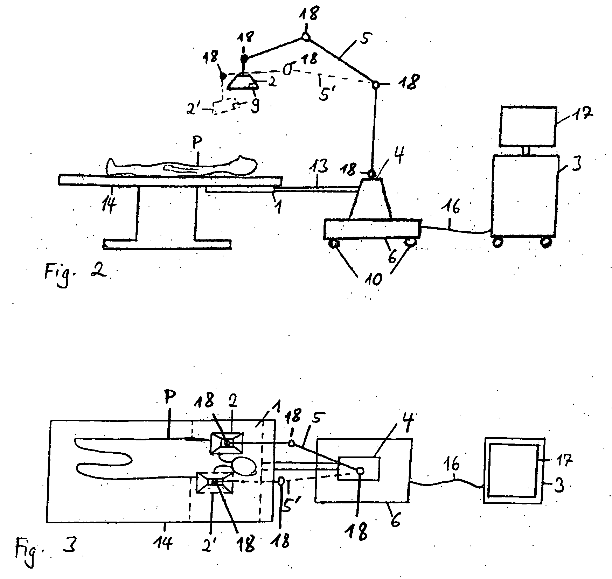

Fig. 2 stellt in seitlicher Ansicht ein Röntgensystem dar, bei dem auf der Positioniereinheit 14 ein Patient P aufliegt. Der Kalibriervorgang ist bereits abgeschlossen und der Kalibrierkörper 15 entfernt. Der Strahlendetektor 1 ist unterhalb der Positioniereinheit angebracht und über einen Adapter 13 mit dem Stativ 4 mechanisch und elektrisch verbunden. Der Adapter kann aufgrund der höheren Stabilität aus Metall sein, aber auch einfach aus Kunststoff, und die elektrische Verbindung 16 zwischen Strahlendetektor 1 und Stativ 4 aufnehmen. Die Positioniereinheit 14 besteht in der dargestellten Anordnung aus Material, das für Röntgenstrahlen durchlässig ist, beispielsweise Kunststoffe. Der Stativfuß 6 ist über blockierbare Räder 10 ortsfest fixiert. Zum Aufnehmen mehrerer Röntgenprojektionsbilder ist der Stativarm 5 und der Röntgenstrahler 2 in unterschiedliche Positionen 2' und 5' verfahren, wobei der Röntgenstrahler 2' gegenüber der ursprünglichen Orientierung verkippt wurde. Die Orientierung und Position des Röntgenstrahlers wird über die Gelenkstellungen des Stativarms bestimmt. Um die Genauigkeit der Orientierungsbestimmung zu erhöhen, enthält der Röntgenstrahler im gezeigten Ausführungsbeispiel zusätzlich einen Lagesensor 9. Der Lagesensor 9 ist ein Beschleunigungssensor, der die Richtung der Erdbeschleunigung als Referenzrichtung auffasst und Verstellungen der Orientierung relativ dazu registriert. Das Stativ 4 ist wie bereits in Figur 1 durch eine elektrische Verbindung 16 mit der Steuereinheit 3 und der Anzeigeeinheit 17 verbunden. Die Datenübertragung zwischen Strahlendetektor, Stativ und Steuereinheit kann auch drahtlos per Funk oder Infrarotschnittstellen geschehen, in diesem Fall wird nur die Stromversorgung über Kabel gewährleistet.

Die in den Figuren 1 und 2 dargestellte Vorrichtung wird wie folgt bedient: ein Kalibrierkörper 15 wird zunächst zwischen Röntgenstrahler 2 und Strahlendetektor 1 eingebracht. Es wird mindestens eine Projektionsaufnahme des Kalibrierkörpers 15 erstellt, aus der zum Beispiel eine Look-Up-Tabelle (LUT) als Kalibriertabelle errechnet und in einem Speicher abgelegt wird. Da der Kalibrierkörper 15 fest am Strahlendetektor 1 angeordnet ist, kann aus dem aufgenommenen Projektionsbild eindeutig auf die Lage und Ausrichtung des Röntgenstrahlers geschlossen werden. Der Kalibrierschritt muss nur einmal vor einer Anwendung durchgeführt werden und erlaubt beliebig viele Aufnahmen von Projektionsbildern, solange das Stativ 4 und der Strahlendetektor 1 raumfest angeordnet bleiben.

Nach Entfernung des Kalibrierkörpers 15 wird an dessen Position das eigentlich abzubildende Objekt eingebracht. Der Röntgenstrahler 2 wird nun in bestimmte Relativpositionen verfahren und in diesen Relativpositionen jeweils ein Projektionsbild aufgenommen. Zur Bildrekonstruktion kann aus jedem Röntgenprojektionsbild zusammen mit den aus der Kalibrierung bekannten Koordinaten des Röntgenstrahlers eine Rekonstruktion des gescannten Volumens erfolgen.

Bei Bedarf kann der Stativarm 5 auch aus dem über der Positioniereinheit 14 befindlichen Arbeitsbereich gefahren werden und zur Aufnahme weiterer Bilder durch einen Motor wieder in die vorherige Position gebracht werden.

Zum Abfahren ständig identischer Relativpositionen wird der Strahlendetektor 2 manuell oder motorisch durch Eingabe der Koordinaten in die Steuereinheit 3 in diese Relativpositionen gefahren und die Koordinaten abgespeichert. Durch ein solches "Teach-in"-Verfahren werden exakte Relativpositionen durch einmaliges Anfahren vorgegeben, die anschließend vom System immer wieder abgefahren werden können.

Fig. 3 zeigt das Röntgensystem der Figur 2 in Aufsicht. Der Strahlendetektor 1 weist die gleiche Breite wie die Positioniereinheit 14 auf, um einen aufliegenden Patienten P bei den Projektionsaufnahmen komplett zu erfassen. Der Stativarm 5 und der Röntgenstrahler 9 sind in alle drei Raumrichtungen bewegbar, wie anhand der Positionen 2' und 5' gezeigt. Die Bewegung kann dabei über mehrere am Stativarm 5 angebrachte Gelenke 18 bewirkt werden. Der Röntgenstrahler 2 ist in sechs Freiheitsgraden, drei translatorischen und drei rotatorischen, bewegbar. Statt eines Patienten P zur medizinischen Röntgenanwendung kann natürlich auch ein anderes Objekt, beispielsweise im Rahmen materialwissenschaftlicher Untersuchungen, mit dem System untersucht werden.

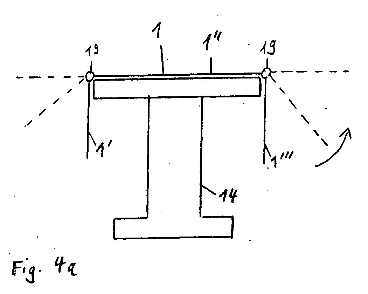

Ein zusammenklappbarer Strahlendetektor 1 ist in Figur 4a dargestellt. Der Strahlendetektor 1 besteht hierbei aus mehreren Detektorplatten 1', 1", 1"', die über eine mechanische Kopplung 19, wie ein Scharnier oder Gelenk, miteinander in Verbindung stehen. Jede der Detektorplatten 1', 1", 1"' besteht dabei aus einem Detektor mit Szintillationsschicht, die, wie bereits beschrieben, Röntgenstrahlung in sichtbares Licht umsetzt, wobei das sichtbare Licht auf einer Pixelmatrix detektiert wird. Die Detektorplatten 1', 1", 1"' sind dabei auch elektrisch gekoppelt über die jeweiligen Verbindungselemente, alternativ können von jeder Platte separate elektrische Leitungen 16 zum restlichen System gehen, die der Übersichtlichkeit halber aber nicht gezeigt sind. Der Strahlendetektor 1 liegt auf einer Positioniereinheit 14 auf. Durch Ausklappen der Detektorplatten 1', 1", 1"' wird der Strahlendetektor 1 auf seine maximale Breite ausgefahren und Röntgenaufnahmen in einem größeren Winkelbereich als bei alleiniger Verwendung 127EP 2021

der Platte 1" sind möglich. Muss am abzubildenden Objekt, das im Betriebszustand auf dem Strahlendetektor 1 aufliegt, etwas verändert werden, so können zum verbesserten Zugang die Platten 1' und 1'" nach unten geklappt werden. Die Bewegung der Platten ist in Fig. 4a durch die gestrichelten Linien verdeutlicht.

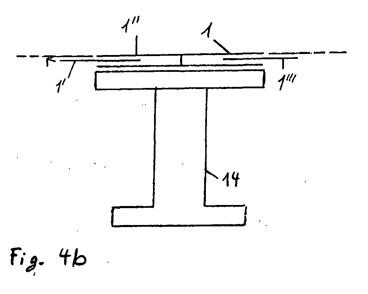

Fig. 4b stellt einen Strahlendetektor 1 mit ausziehbaren Detektorplatten 1', 1", 1"' dar. Die Platten 1' und 1'" lassen sich, sofern sie nicht benötigt werden, unter die Platte 1" versenken, sodass der Strahlendetektor 1 eine kompakte und leicht zu transportierende Einheit bildet. Der Strahlendetektor 1 liegt wiederum auf der Positioniereinheit 14 auf, kann aber einfach von dieser entfernt werden. Die ausgezogene Position der Platten 1' und 1"' ist wiederum durch gestrichelte Linien angedeutet. Auch in dieser Ausgestaltung sind die einzelnen Platten 1', 1", 1"' mechanisch und elektrisch miteinander in Kontakt. Bei fehlendem elektrischem Kontakt gehen hier nicht gezeigte elektrische Verbindungen 16 zum Stativ 4 und/oder der Steuereinheit.

Ein Aspekt der Erfindung betrifft ein Röntgensystem nach einem der Ansprüche 1 bis 6, das dadurch gekennzeichnet ist, dass der Strahlendetektor 1 durch Haltevorrichtungen 12 raumfest lösbar positionierbar ist und vorzugsweise an raumfesten Objekten befestigbar ist.

Außerdem ist ein auf das Röntgensystem des vorherigen Aspekts bezogener Aspekt der Erfindung dadurch gekennzeichnet, dass der Stativarm 5 auch manuell bewegbar ist.

Ein Aspekt der Erfindung betrifft das Röntgensystem der beiden vorhergehenden Aspekte und ist dadurch gekennzeichnet, dass der Strahlendetektor 1 durch einen Adapter 13 mechanisch mit dem Stativ 4 verbunden ist.

Ein weiterer Aspekt der Erfindung betreffend das Röntgensystem nach einem der vorherigen Aspekte ist dadurch gekennzeichnet, dass der Strahlendetektor 1 als klappbares oder ausziehbares Mehrkomponentensystem mehrerer einzelner Detektorplatten 1, 1', 1" ausgebildet ist.

Ein Aspekt der Erfindung betrifft ein Verfahren zur Benutzung eines Röntgensystems nach dem Hauptanspruch, welches die folgenden Schritte umfasst:
a) Kalibrieren des Systems bei raumfest fixiertem Strahlendetektor 1 und raumfest fixiertem Stativfuß 6 durch einen zwischen Röntgenstrahler 2 und Strahlendetektor 1 befindlichem Kalibrierkörper 15, wobei mindestens ein Röntgenprojektionsbild des Kalibrierkörpers 15 aufgenommen wird,
b) Aufnehmen von Röntgenprojektionsbildern eines sich zwischen Röntgenstrahler 2 und dem Strahlendetektor 1 befindlichen abzubildenden Objekts,
c) Berechnen der 3D-Rekonstruktion des gescannten Volumens durch eine iterative Rekonstruktionstechnik oder andere Bildverarbeitungsmethoden aus den Röntgenprojektionsbildern des abzubildenden Objekts und den Koordinaten der zum Aufnehmen verwendeten Relativposition.

Ein weiterer Aspekt der Erfindung betrifft das Verfahren nach dem vorherigen Aspekt und ist dadurch gekennzeichnet, dass einem motorisch bewegbaren Stativarm 5 durch ein "Teach-in"-Verfahren auf einer Aufnahmebahn zur Aufnahme von Röntgenprojektionsbildern liegende Relativpositionen vorgegeben werden und die Steuereinheit 3 die Relativposition dieser Aufnahmebahn automatisch abfahren kann.

Ein weiterer Aspekt betrifft das Verfahren der beiden vorhergehenden Aspekte und ist dadurch gekennzeichnet, dass auf einer durch ein "Teach-in"-Verfahren vorgegebenen Aufnahmebahn durch eine Auswertung einer vordefinierten Anzahl von Röntgenprojektionsbildern eines abzubildenden Objekts die optimalen Aufnahmepositionen des Röntgenstrahlers 2 auf der Aufnahmebahn berechnet werden und die Steuereinheit 3 für zukünftige Aufnahmen diese berechneten optimalen Relativpositionen abfährt.

### Bezugszeichenliste:

- 1: Strahlendetektor
- 1': Detektorplatte
- 1": Detektorplatte
- 1"': Detektorplatte
- 2: Röntgenstrahler
- 2': Röntgenstrahler in veränderter Position
- 3: Steuereinheit
- 4: Stativ
- 5: Stativarm
- 5': Stativarm in veränderter Position
- 6: Stativfuß
- 7: Sensoren zur Abstandsmessung
- 8: Bewegungsmelder
- 9: Lagesensor
- 10: blockierbare Räder
- 11: Vakuumansaugsystem
- 12: Haltevorrichtungen
- 13: Adapter
- 14: Positioniereinheit
- 15: Kalibrierkörper
- 16: elektrische Verbindungen
- 17: Anzeigeeinheit
- 18: Gelenke
- 19: Kopplung

- P: Patient
- L: Laserstrahl

## Patentansprüche

1. Röntgensystem zur Generierung von 3D-Bilddaten, einen Strahlendetektor (1), einen beweglichen Röntgenstrahler (2) und eine Steuereinheit (3) umfassend, wobei der Strahlendetektor (1) in einem Arbeitszustand des Systems raumfest lösbar positioniert ist und ein ortsfestes Basiskoordinatensystem definiert und der Röntgenstrahler (2) zur Aufnahme von Röntgenprojektionsbildern relativ zum Strahlendetektor (1) in unterschiedliche Relativpositionen bewegbar ist, wobei in den Relativpositionen aufgenommene Projektionsbilder in Kombination mit den Koordinaten der Relativpositionen durch eine iterative Rekonstruktionstechnik oder andere Bildverarbeitungsmethoden zu 3D-Röntgenaufnahmen verarbeitbar sind, und wobei das System ein bewegliches Stativ (4) mit einem motorisch beweglichen Stativarm (5) und einem Stativfuß (6) umfasst, wobei der Röntgenstrahler (2) am Stativarm (5) befestigt und in mindestens vier Freiheitsgraden beweglich ist und der Stativfuß (6) Befestigungsvorrichtungen zum raumfesten Positionieren aufweist und im Arbeitszustand raumfest derart positioniert ist, dass er eine deflnierte feste Position innerhalb des Basiskoordinatensystems aufweist und dass der Stativarm (5) durch die Steuereinheit (3) in die Relativpositionen verfahrbar ist, wobei der Stativfuß (6) Sensoren (7) zur Überprüfung einer Beibehaltung einer raumfesten Positionierung des Stativfußes (6) umfasst.

2. Röntgensystem nach einem der vorhergehenden Ansprüche, **dadurch gekennzeichnet, dass** zur Prüfung, ob sich während einer Röntgenaufnahme Personen an bestimmten Stellen befinden, Sensoren (8) enthalten sind, welche bei Vorhandensein von Personen ein Signal an die Steuereinheit (3) zum Unterbrechen der Röntgenaufnahme übermitteln.

3. Röntgensystem nach einem der Ansprüche 1 oder 2, **dadurch gekennzeichnet, dass** die Sensoren Laser umfassen.

4. Röntgensystem nach einem der vorhergehenden Ansprüche, **dadurch gekennzeichnet, dass** der Röntgenstrahler (2) in fünf, vorzugsweise sechs, Freiheitsgraden bewegbar ist.

5. Röntgensystem nach einem der vorhergehenden Ansprüche, **dadurch gekennzeichnet, dass** Lagesensoren (9) zum Ermitteln der Relativlage des Röntgenstrahlers (2) bezüglich des Basiskoordinatensystems enthalten sind.

6. Röntgensystem nach einem der vorhergehenden Ansprüche, **dadurch gekennzeichnet, dass** die Befestigungsvorrichtungen zum raumfesten Positionieren des Stativfußes (6) blockierbare Räder (10) oder ein Vakuumansaugsystem (11) umfassen.

7. Röntgensystem nach einem der vorhergehenden Ansprüche, **dadurch gekennzeichnet, dass** eine Positioniereinheit (14) enthalten ist, wobei die Positioniereinheit (14) und der Strahlendetektor (1) vorzugsweise eine gleiche Breite aufweisen.

## Claims

1. An X-ray system for generating 3D image data, comprising a radiation detector (1), a movable X-ray tube assembly (2) and a control unit (3), wherein in a working state of the system the radiation detector (1) is detachably positioned in a spatially fixed manner and defines a locally fixed base coordinate system, and the X-ray tube assembly (2) can be moved relative to the radiation detector (1) into different relative positions so as to record X-ray projection images, wherein projection images recorded in the relative positions, combined with the coordinates of the relative positions, can be processed using an iterative reconstruction technique or other image processing methods to obtain 3D X-ray images, and wherein the system comprises a movable stand (4) having a motor-driven movable stand arm (5) and a stand base (6), wherein the X-ray tube assembly (2) is attached to the stand arm (5) and can be moved in at least four degrees of freedom, and the stand base (6) comprises fastening devices for the spatially fixed positioning and, in the working state, is positioned in a spatially fixed manner such that it has a defined fixed position within the base coordinate system, and the stand arm (5) can be displaced into the relative positions by way of the control unit (3), wherein the stand base (6) comprises sensors (7) for controlling that a spatially fixed position of the stand base (6) is maintained.

2. The X-ray system according to one of the preceding claims, **characterized in that** sensors (8) are provided for monitoring particular areas for the presence of people during an X-ray recording procedure, which transmit a signal to the control unit (3) so as to interrupt the X-ray recording if people are present.

3. The X-ray system according to one of claims 1 or 2, **characterized in that** the sensors comprise lasers.

4. An X-ray system according to one of the preceding claims, **characterized in that** the X-ray tube assembly (2) can be moved in five, and preferably in six, degrees of freedom.

5. An X-ray system according to one of the preceding claims, **characterized in that** position sensors (9) are provided for determining the relative position of the X-ray tube assembly (2) with respect to the base coordinate system.

6. An X-ray system according to one of the preceding claims, **characterized in that** the fastening devices comprise blockable wheels (10) or a vacuum suction system (11) for positioning the stand base (6) in a spatially fixed manner.

7. An X-ray system according to one of the preceding claims, **characterized in that** a positioning unit (14) is present, the positioning unit (14) and the radiation detector (1) preferably having the same width.

## Revendications

1. Système à rayons X pour la génération de données d'images 3D, comprenant un détecteur de rayonnement (1), un émetteur de rayons X (2) mobile et une unité de commande (3), dans lequel détecteur de rayonnement (1) est positionné de manière immobile réversible dans un état de fonctionnement du système et définit un système de coordonnées de base fixes et l'émetteur de rayons X (2) peut être déplacé relativement par rapport au détecteur de rayonnement (1) dans des positions relatives différentes pour la prise d'images de projection par rayons X, des images de projection prises dans les positions relatives, en combinaison avec les coordonnées des positions relatives, peuvant être traitées pour donner des prises de vue par rayons X en 3D par une technique de reconstruction itérative ou d'autres procédés de traitements d'images, et lequel système comprend un support (4) mobile avec un bras de support (5) motorisé mobile et un pied de support (6), l'émetteur de rayons X (2) étant fixé sur le bras de support (5) et étant mobile dans au moins quatre degrés de liberté, et le pied de support (6) présentant des dispositifs de fixation pour le positionnement immobile et étant positionné dans la position de fonctionnement de manière immobile dans l'espace de telle manière qu'il présente une position fixe définie dans le système de coordonnées de base et que le bras de support (5) peut être déplacé dans les positions relatives par l'unité de commande (3), où le pied de support (6) comprenant des capteurs (7) pour le contrôle de la conservation d'un positionnement immobile dans l'espace du pied de support (6).

2. Système à rayons X selon l'une des revendications précédentes, **caractérisé en ce que**, pour la vérification si des personnes se trouvent à des localisations déterminées pendant une prise de vue par les rayons X, des capteurs (8) sont inclus, lesquels transmettent un signal vers l'unité de commande (3) pour une interruption de la prise de vue par les rayons X lors d'une présence de personnes.

3. Système à rayons X selon l'une des revendications 1 ou 2, **caractérisé en ce que** les capteurs comprennent des lasers.

4. Système à rayons X selon l'une des revendications précédentes, **caractérisé en ce que** l'émetteur de rayons X (2) peut être déplacé dans cinq, de préférence, six, degrés de liberté.

5. Système à rayons X selon l'une des revendications précédentes, **caractérisé en ce que** des capteurs de position (9) sont inclus pour l'évaluation de la position relative de l'émetteur de rayons X (2) par rapport au système de coordonnées de base.

6. Système à rayons X selon l'une des revendications précédentes, **caractérisé en ce que** les dispositifs de fixation comprennent des roues (10) pouvant être bloquées ou un système d'aspiration sous vide (11) pour le positionnement immobile dans l'espace du pied de support (6).

7. Système à rayons X selon l'une des revendications précédentes, **caractérisé en ce qu'**une unité de positionnement (14) est incluse, où l'unité de positionnement (14) et le détecteur de rayonnement (1) présentent de préférence une largeur identique.
